# EUROPEAN PATENT SPECIFICATION

(11) **EP 2 608 094 B1**
(45) Date of publication and mention of the grant of the patent: **11.10.2017**
(21) Application number: 12198718.4
(22) Date of filing: 20.12.2012
(51) Int. Cl.: G06F 19/00

(54) **Medical apparatus and image displaying method using the same**
Medizinische Vorrichtung und Bildanzeigeverfahren damit
Appareil médical et procédé d'affichage d'image l'utilisant

(30) Priority: 21.12.2011 KR 20110139025
(43) Date of publication of application: 26.06.2013
(73) Proprietor: Samsung Electronics Co., Ltd., Gyeonggi-do 443-742 (KR)
(72) Inventor: Lee, Byeong Won, Gyeonggi-do (KR)
(74) Representative: Grootscholten, Johannes A.M.

(56) References cited:
- WO-A2-2008/064120
- WO-A2-2011/044295
- US-A1- 2002 094 119
- "PhotoImpact 10 User Guide" In: "PhotoImpact 10 User Guide", 1 August 2004 (2004-08-01), Ulead Systems Inc., XP055057026, pages 1-366, * page 30 - page 32 * * page 42 - page 45 * * page 52 - page 53 * * page 59 * * page 87 - page 96 *

## Description

### BACKGROUND

### 1. Field

Exemplary embodiments relate to a medical apparatus which displays images processed through image processing, and an image display method using the same.

### 2. Description of the Related Art

In general, clinical diagnosis in medical behavior is a major part of treatment of a patient, development of medical techniques facilitates precise clinical diagnosis, and the degree of dependence thereon will continue to increase in the future.

Therefore, medical image equipment, such as a computer tomography (CT) apparatus, a magnetic resonance imager (MRI), an X-ray apparatus and an ultrasonic apparatus, have become essential equipment in modern medical science.

However, since a medical service allows an abnormal region of a patient to be photographed using medical image equipment and then printed on a film. The film must be transmitted to a doctor in charge of the patient. Thus, the medical service has an ineffective structure in that it takes considerable time and a lot of manpower to finally make a clinical diagnosis and causes difficulty in performing rapid and precise treatment of the patient.

Further, as computer and data communication technology develop, a system of providing a medical service using the computer and data communication technology has been researched and developed in the medical community. For example, a medical image system in which a computer communication network is installed throughout the entirety of a hospital, all X-ray films are converted into digital data, a database of the digital data is stored in a large storage medium connected to a server, and an X-ray image of a desired patient is searched through a computer monitor in each medical office as needed, has been introduced.

In order to provide better quality images by removing artifacts, generated due to the physical structure of the medical image equipment, from images acquired through medical image equipment, image processing is needed. US-2002/0094119 discloses manual processing by trained technicians to provide a single processed image in addition to an original analogue image from film.

Since such image processing by medical staff is important to more precisely and rapidly make a diagnosis, development of a user interface used to perform image processing more conveniently and intuitively is required. Document US2002/094119 (Sahadevan Velayudhan [US], 18 July 2002) is a system for processing medical images, including image adjustments (contrast, brightness, gamma, etc.) with the purpose of easy detection of lesions, tumours, etc., and separate image processing.

Document XP055057026 (Photoimpact 10 User Guide, Ulead systems Inc., 1 August 2004) discloses a commercial software for image processing, allowing adjustments about colours brightness, contrast, gamma, grid of thumbnails for previewing transformations.

Document WO2011/044295 (Hologic Inc; Kreeger Kevin A; marchall Julian; Hitzke G, 14 April 2011) allows basic image transformations, thumbnails for previews, for radiology (mammographies).

Document WO2008/064120 (Amigent Inc; Bergman Harris L, 29 May 2008) is a medical image processing system disclosing image transformations (colour, contrast adjustments), contour/border detection of lesions, regions of interest.

### SUMMARY

Therefore, according to an aspect of the invention, an exemplary embodiment provides a medical image processing apparatus which displays additional images to which various types of image processing is applied, and an image displaying method using the same.

Additional aspects of the exemplary embodiments will be set forth in part in the description which follows and, in part, will be obvious from the description, or may be learned by implementing the exemplary embodiments.

In accordance with an aspect of an exemplary embodiment, a medical apparatus includes all features of the appended independent medical image processing apparatus.

When instructions to an object are input, the controller may magnify and display the processed image represented by the object on the display unit.

The medical apparatus may further include an input unit (e.g., an input, an input device, etc.) including a remote controller, a mouse, a keyboard, a speech recognition device or a motion recognition device, and the instructions to the object may be input through the input unit.

The display unit may comprise a touchscreen, and the instructions to the object may be input by touching the object displayed on the display unit.

The objects may include thumbnail pictures of the images processed through the various or multiple predetermined types of image processing or text representing features of the various predetermined types of image processing.

The various or multiple predetermined types of image processing may include post-processing of an image having undergone pre-processing.

The display unit may display the overall image of the subject, information regarding the image, a list of viewable images, tools to process the image and the objects of the images processed by the various or multiple predetermined types of image processing, and display, if instructions to an object are input, the processed image represented by the object.

In accordance with another aspect of an exemplary embodiment, a control method of a medical apparatus includes all feature steps of the appended independent method claim.

When instructions to an object are input, the processed image represented by the object may be magnified and displayed on the display unit.

The medical apparatus may include an input unit including a remote controller, a mouse, a keyboard, a speech recognition device or a motion recognition device, and the instructions to the object may be input through the input unit.

The display unit may use a touchscreen, and the instructions to the object may be input by touching the object displayed on the display unit.

The objects may include thumbnail pictures of the images processed through the various or multiple predetermined types of image processing or text representing features of the various or multiple predetermined types of image processing.

When the image of the subject is received, the overall image of the subject may be displayed.

The objects together with the overall image of the subject may be displayed on the display unit of the medical apparatus.

The processing of the image through various or multiple predetermined types of image processing, when the image of the subject is received, may include pre-processing the image, when the image of the subject is received, and post-processing the image through the various or multiple predetermined types of image processing, separately from the processing of the image.

In accordance with another aspect of an exemplary embodiment, there is provided a non-transitory computer readable recording medium in which a program implementing the control method is recorded.

In accordance with another aspect of an exemplary embodiment, a control method of a medical apparatus includes processing an image of a subject, when the image of the subject is received, and displaying the processed image on a display unit of the medical apparatus, respectively processing the image through various or multiple predetermined types of image processing and displaying objects corresponding to the processed image on the display unit, during the processing of the image separately from the processing of the image, and when instructions to an object are input, magnifying and displaying the processed image represented by the object on the display unit.

The display unit may use a touchscreen, and the instructions to the object may be input by touching the object displayed on the display unit.

The medical apparatus may include an input unit including a remote controller, a mouse, a keyboard, a speech recognition device or a motion recognition device, and the instructions to the object may be input through the input unit.

The objects may include thumbnail pictures of the images processed through the various or multiple predetermined types of image processing or texts representing features of the various predetermined types of image processing.

The various or multiple predetermined types of image processing may include post-processing of an image having undergone pre-processing.

The post-processing may adjust at least one of parameters of the image having undergone pre-processing, including brightness, contrast, sharpness, saturation, film-effect and window/level.

In accordance with a further aspect of an exemplary embodiment, there is provided a non-transitory computer readable recording medium in which a program implementing the control method is recorded.

In accordance with an aspect of an exemplary embodiment, a control method of a medical apparatus includes: processing an image of a subject through multiple predetermined types of image processing and displaying the processed image on a display of the medical apparatus; generating and displaying an object corresponding to the processed image on the display; and in response to inputting instructions into the object, magnifying and displaying the processed image represented by the object on the display.

The image of the subject may include at least one from among an MRI image, a CT image, an X-ray image, and an ultrasonic image.

The processing of the image of the subject may adjust at least one of parameters of the image having undergone pre-processing, including brightness, contrast, sharpness, saturation, film-effect and window/level.

### BRIEF DESCRIPTION OF THE DRAWINGS

These and/or other aspects of the exemplary embodiments will become apparent and more readily appreciated from the following description of the embodiments, taken in conjunction with the accompanying drawings of which:
FIGS. 1 and 2 are views illustrating the configuration of a medical image system according to an aspect of an exemplary embodiment;
FIGS. 3 and 4 are views illustrating the configuration of a user interface according to an aspect of an exemplary embodiment;
FIG. 5 is a view illustrating the user interface according to an aspect of an exemplary embodiment; and
FIG. 6 is a flowchart illustrating an image displaying method according to an aspect of an exemplary embodiment.

### DETAILED DESCRIPTION OF EXEMPLARY EMBODIMENTS

Reference will now be made in detail to the exemplary embodiments, which are illustrated in the accompanying drawings, wherein like reference numerals refer to like elements throughout the description.

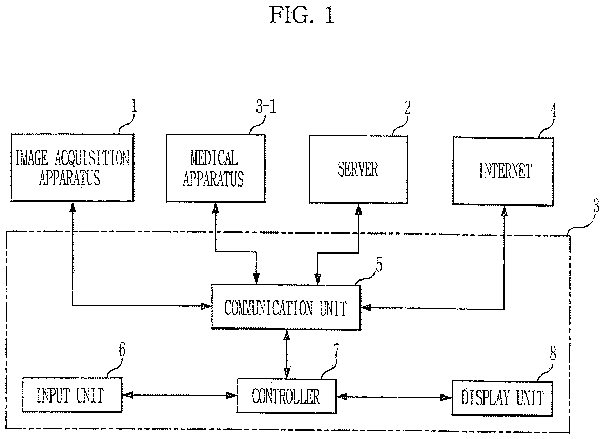

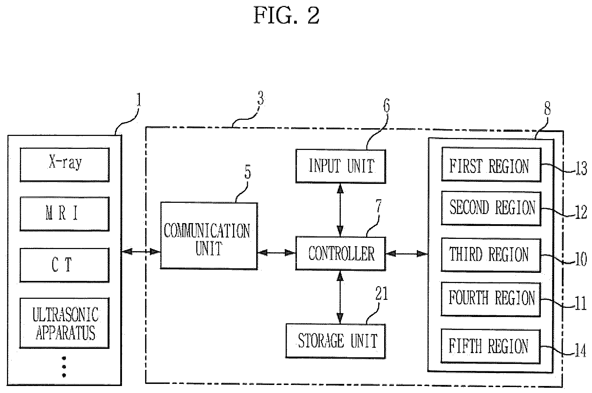

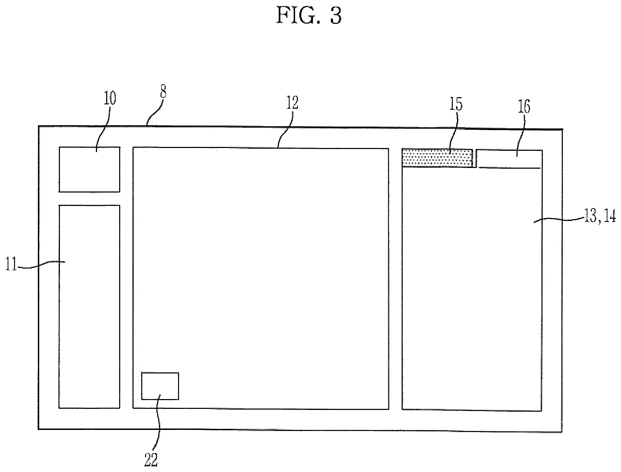

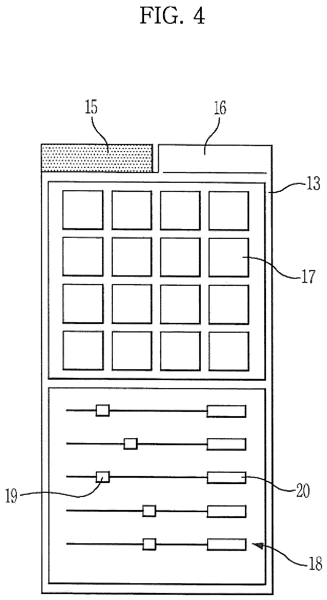

FIGS. 1 and 2 are views illustrating the configuration of a medical image system according to an aspect of an exemplary embodiment, and FIGS. 3 and 4 are views illustrating the configuration of a user interface according to an aspect of an exemplary embodiment.

As shown in FIG. 1, the medical image system includes an image acquisition apparatus 1 acquiring an image of a subject, for example, a patient, and a medical apparatus 3 processing the image processed by the image acquisition apparatus 1 and displaying the processed image.

The medical apparatus 3 is a workstation controlling the overall operation of the medical image system, processes the image processed by the image acquisition apparatus 1, and displays the processed image.

Further, the medical apparatus 3 may be a picture archiving communication system (PACS) viewer which receives the image processed by the image acquisition apparatus 1 from a server 2 or receives the image directly from the image acquisition apparatus 1, processes the received image, and displays the processed image.

The medical apparatus 3 includes a communication unit 5 (e.g., a communicator, transceiver, receiver, receiving unit, etc.) receiving image information of the subject from the image acquisition apparatus 1 through communication with the image acquisition apparatus 1, an input unit 6 providing an interface for operation of the medical apparatus 3, a controller 7 controlling the overall operation of the medical image system including the medical apparatus 3 and the image acquisition apparatus 1 according to instructions input through the input unit 6 and processing the image of the subject received from the communication unit 5, a storage unit 21 storing the processed image and various pieces of information, and a display unit 8 (e.g., a display, etc.) displaying the state of the medical apparatus 3 or the image acquisition apparatus 1 and the various pieces of information and displaying the image processed by the controller 7.

Such a medical apparatus 3 may include a PC, a portable smart-phone or a tablet PC, and a PACS viewer supporting a digital imaging and communication in medicine (DICOM) protocol.

As shown in FIG. 2, the image acquisition apparatus 1 acquires the image of a subject, for example, a patient, and is an MRI, a CT apparatus, an X-ray apparatus or an ultrasonic apparatus. The image of the subject may be at least one from among an MRI image, a CT image, an X-ray image, and an ultrasonic image.

The image acquisition apparatus 1 acquires the image of the subject, and particularly, acquires the image of the inside of the subject, according to the function thereof.

Image data of the subject may be based on the digital imaging and communication in medicine (DICOM) protocol which is a standard protocol.

Although this embodiment describes the communication unit 5 as receiving the image of the subject from the image acquisition apparatus 1, the communication unit 5 may receive various medical images from a server 2 in figure 1 in which images processed by various image acquisition apparatuses are sorted and stored, or may receive medical images via the Internet 4 or from other computers or other medical apparatuses 3-1.

Further, the communication unit 5 may transmit control instructions to control the operation of the image acquisition apparatus 1 from the controller 7 to the image acquisition apparatus 1, or may transmit various pieces of information modified or processed by the controller 7 to the server 2, the Internet 4, or other medical apparatuses 3.

Here, the server 2 may be a PACS server supporting the DICOM protocol.

The communication unit 5 may employ various known wired and wireless communication methods in communication with external devices.

The input unit 6 provides the interface so as to allow a user of the medical apparatus 3, for example, medical staff, to operate the overall function of the medical image system including the medical apparatus 3 and the image acquisition apparatus 1, and may include a remote controller, a mouse, a keyboard, a speech recognition device or a motion recognition device.

The medical staff may properly operate the overall function of the medical image system, as needed, through peculiar functions of the above-described various input devices.

If the display unit 8 uses a touchscreen, a user may operate the function of the medical apparatus 3 by directly touching various icons displayed on the display unit 8. That is, the display unit 8 may perform the function of the input unit 6 in addition to the function of displaying various pieces of information.

The display unit 8 provides a user interface, and the user interface may include a plurality of regions in which windows of various sizes and shapes having peculiar functions are displayed.

The user interface according to an aspect of an exemplary embodiment provides information regarding processing of the image of a subject, i.e., image processing, and provides environments allowing a user to perform operations related to image processing.

With reference to FIGS. 2 and 3, the user interface provided by the display unit 8 includes a first region 13 displaying objects 17 of respective images processed through various predetermined kinds or types of image processing, a second region 12 displaying an image processed through image processing, a third region 10 displaying information regarding the image of a subject, a fourth region 11 displaying a list of selectable image files, and a fifth region 14 providing image processing tools to process the image displayed in the second region 12.

The third region 10 may display the information regarding the subject. On the assumption that the subject is a patient, the third region 10 displays information, such as personal information and disease history of the patient, so that a user may refer to such information.

The fourth region 11 may display a list of image files viewable by the user. The fourth region 11 may display images transmitted from other medical apparatuses 3, the Internet 4 and the server 2, as described above, as well as the image transmitted from the image acquisition apparatus 1.

In the fourth region 11, the images are classified based on the devices or media transmitting the images, and may thus be sorted and displayed according to a device or medium selected by the user.

When the user touches or clicks an image file desired to view from among the image files displayed on the list displayed in the fourth region 11, the corresponding image file is displayed in the fourth region 11, and thus the user may confirm the image of the selected file.

The fifth region 14 displays the image processing tools so as to allow the user to process the image displayed in the second region 12 as desired.

For example, the fifth region 14 may display a tool for adjusting the size of an image displayed on the display unit 8, a tool for rotating the image, a tool for cutting out a desired portion of the image, a tool for magnifying or reducing a desired portion of the image, etc.

These tools may be expressed by symbolized icons intuitively representing the functions of the tools. If the display unit 8 uses a touchscreen, the user may touch the icon expressing a designated tool or click the icon through the input unit 6, thus being capable of processing the image according to the function of the corresponding tool. When the image is processed by the tool selected by the user, the processed image is displayed in the second region 12.

The first region 13 may be designed to possess the same area on the user interface in common with the fifth region 14, and the user may select the first region 13 or the fifth region 14 providing desired contents through tabs 15 and 16 to select the first region 13 and the fifth region 14, displayed on the upper portion of the corresponding area. Although the first region 13 and the fifth region 14 may be displayed in separate areas, since the first region 13 and the fifth region 14 provide contents executing similar functions for the purpose of image processing, the first region 13 and the fifth region 14 may be displayed in the same area so as to allow the user to more easily understand the configuration of the interface. Further, the tabs 15 and 16 are provided so as to allow the user to select the contents displayed in the corresponding area, and thus the user interface may be designed to have a simpler structure.

The second region 12 displaying the image processed through image processing and the first region 11 displaying objects 17 of respective images processed through various predetermined kinds of image processing will be described later with reference to image processing.

The controller 7 controls the overall operation of the medical image system including the medical apparatus 3 and the image acquisition apparatus 1 according to information received through the communication unit 5 or external instructions input through the input unit 6 or the display unit 8.

The controller 7 may control the display unit 8 so as to display a medical image or a diagnosis record, and, if the medical image or the diagnosis record is amended or updated, may store the amended or updated medical image or diagnosis record in the storage unit 21, and may transmit the amended or updated medical image or diagnosis record to the server 2 through the communication unit 5 so as to store the amended or updated medical image or diagnosis record in the server 2.

The controller 7 basically performs image processing to provide an image having a more improved quality, when the controller 7 receives the image of the subject transmitted from the image acquisition apparatus 1 through the communication unit 5.

Image processing may be divided into pre-processing and post-processing.

Pre-processing is a process of removing artifacts or errors generated due to the physical structure of the image acquisition apparatus 1, and post-processing is a process of improving the quality of an image having undergone pre-processing and during post-processing, contrast, sharpness and brightness are determined according to set parameters.

The controller 7 displays the image processed through image processing on the display unit 8. The image having undergone the basic image processing is displayed in the second region 12 of the user interface provided by the display unit 8.

Separately from the above-described image processing basically executed with respect to the received image, the controller 7 executes various predetermined kinds of image processing with respect to the received image. More specifically, the controller 7 executes various kinds of post-processing of the image having undergone pre-processing.

For example, the controller 7 executes various kinds of post-processing exhibiting various effects, such as brightness, contrast, sharpness, saturation, film-effect, window/level, etc., with respect to the image from which the artifacts or errors are removed through pre-processing. Hereinafter, the above-described basic image processing will be referred to as first image processing, and the various kinds of predetermined image processing executed separately from the first image processing will be referred to as second image processing.

That is, when the controller 7 receives an image, the controller 7 amends artifacts or errors of the image through pre-processing, executes first image processing by executing first post-processing with respect to the image having undergone pre-processing, and executes second image processing by respectively executing the above-described various kinds of second post-processing with respect to the image having undergone pre-processing. The various kinds of second post-processing may include all kinds of post-processing which may be executed with respect to the image. Further, the controller 7 may select kinds of post-processing which a user often uses by calculating the use frequencies of the kinds of post-processing selected by the user, and may form second post-processing consisting of the selected kinds of post-processing.

The controller 7 generates images variously processed by executing second image processing, and displays objects 17 of the images in the first region 13 of the display unit 8.

Here, the objects 17 include thumbnail pictures of the images processed by executing various kinds of post-processing and texts expressing features of the respective kinds of post-processing. The text representing the features of a kind of post-processing means, for example, if the kind of post-processing applied to the image is change of contrast, a text expressing the feature of the corresponding kind of post-processing, such as contrast, of the image having undergone the corresponding kind of post-processing. In order to allow a user to intuitively recognize and expect an execution result of post-processing, the objects 17 may be expressed by thumbnail pictures or expressed by both thumbnail pictures and texts.

Second image processing together with first image processing is executed with respect to the image having undergone pre-processing. Differently from first image processing, in second image processing, various kinds of post-processing are respectively executed and various images processed by executing the respective kinds of post-processing are generated, as described above, and thus second image processing may take a longer time than first image processing.

When first image processing is completed, an image processed by executing first image processing is displayed in the second region 12 of the user interface, and if second image processing is not completed, the thumbnail picture of the image is not displayed. Therefore, until second image processing is completed, the objects 17 are expressed by texts in the first region 13, and when second image processing is completed, the texts may be changed to thumbnail pictures, or both the texts and the thumbnail pictures may be displayed. Of course, when second image processing is completed simultaneously with first image processing or is completed prior to first image processing, the objects 17 may be expressed by thumbnail pictures.

Since the respective objects 17 represent images to which various kinds of post-processing are respectively applied, when a user inputs instructions to an object 17, a processed image represented by the object 17 to which the instructions are input is magnified and displayed on the display unit 8.

For example, if a user desires to change the contrast of the image displayed in the second region 12, when the user touches or clicks the thumbnail picture of the image in which contrast is changed from among several thumbnail pictures, the image represented by the corresponding thumbnail picture is magnified and displayed in the second region 12.

In this way, when images processed by executing various kinds of post-processing are represented by the objects 17 allowing a user to intuitively recognize kinds of post-processing to be executed, such as the thumbnail pictures, the user may touch or click a thumbnail picture to confirm an image to which the corresponding kind of post-processing is applied.

The user may only touch or clock a thumbnail picture while confirming an image processed by executing a kind of post-processing regarding a corresponding parameter and expressed by the thumbnail picture in advance without a troublesome operation, such as a manual adjustment of the parameter, and thus may more easily and simply process the image as the user desires.

From the viewpoint of the user interface, the second region 12 displays an image to which first image processing is applied, and the first region 13 displays thumbnail pictures of respective images to which second image processing is applied. When the tab 16 to select the first region 13 from among the first region 13 and the fifth region 14 providing processing tools to process an image, displayed in the same area is touched or clicked, contents provided by the first region 13 is displayed in the corresponding area. That is, the first region 13 displays thumbnail pictures of respective images to which second image processing is applied. When a thumbnail picture to which a desired kind of post-processing is applied is touched or clicked from among these several thumbnail pictures, a processed image represented by the thumbnail picture is magnified and displayed in the second region 12.

A window 22 displaying an image before it is changed into a processed image may be provided in the second region 12, and when the window 22 is touched or clicked, the image before the change into the processed image may be displayed again. That is, the image before the change and the image after the change may be compared and be converted directly therebetween. The window 22 displaying the image before the change may be provided to occupy a small-sized area at the corner of the second region 12 if display of the image after the change is emphasized, and may be provided to occupy the same-sized area or a similar-sized area as or to the image after the change if a comparison between the image before the change and the image after the change is emphasized. This may be adjusted according to a change of setting by a user.

A parameter adjustment window 18 may be provided in the first region 13 so as to be manipulated when a user desires more detailed post-processing. The user may input specific parameter values into value input windows 20 displayed in the parameter adjustment window 18, or may drag icons 19 to adjust parameters.

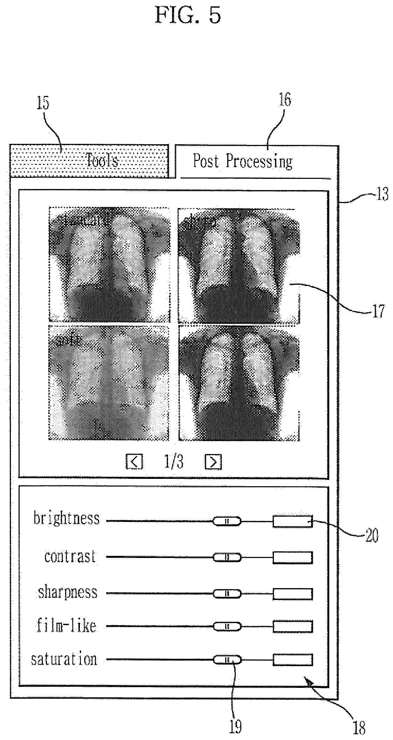

FIG. 5 is a view illustrating the user interface according to an aspect of an exemplary embodiment.

FIG. 5 specifically illustrates the above-described first region 13. When the tab 16 to select the first region 13 is touched or clicked from among the tabs 15 and 16 to select one of the first region 13 and the fifth region 14, contents provided by the first region 13 may be displayed, as shown in FIG. 5.

Thumbnail pictures 17 and texts of images processed by executing various predetermined kinds of image processing, i.e., second image processing, are displayed at the upper portion of the first region 13. As described above, the user may touch or click the thumbnail picture 17 to which a desired kind of image processing is applied from among the displayed thumbnail pictures 17, thus confirming the processed image represented by the corresponding thumbnail picture 17 in the second region 12.

The parameter adjustment window 18 may be displayed at the lower portion of the first region 13 so as to be manipulated when the user desires more detailed post-processing. The user may input specific parameter values into the value input windows 20 displayed in the parameter adjustment window 18, or may drag the icons 19 to adjust parameters.

Although the user interface of FIG. 5 is an example of FIG. 4, the exemplary embodiments not limited thereto and the user interface of FIG. 5 may be modified in various ways.

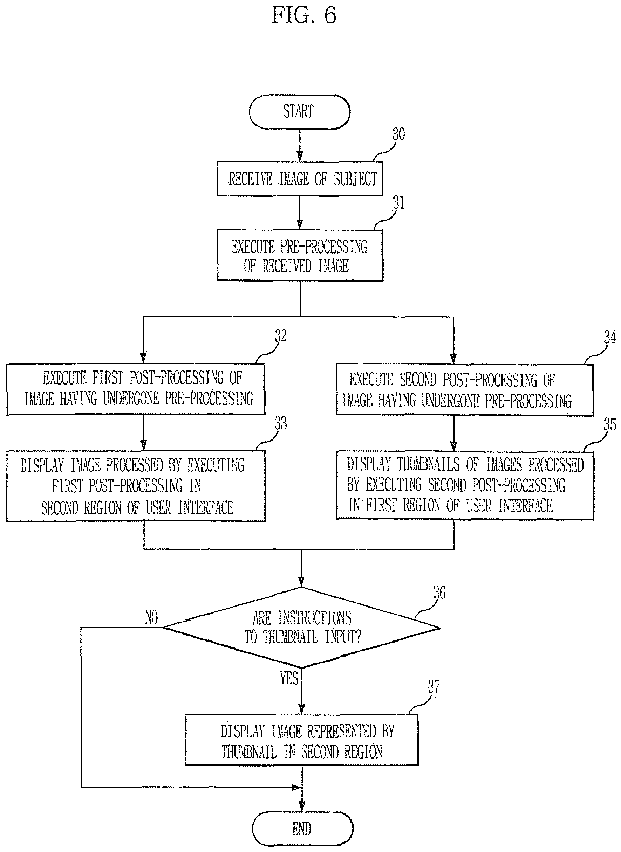

FIG. 6 is a flowchart illustrating an image displaying method according to an aspect of an exemplary embodiment.

With reference to FIG. 6, first, an image of a subject is received (Operation 30).

The communication unit 5 receives the image of the subject from the image acquisition apparatus 1, another medical apparatus 3, the Internet 4 or the server 2.

When the image of the subject is received, the controller 7 executes pre-processing of the received image (Operation 31). Pre-processing is a process of removing artifacts or errors generated due to the physical structure of the image acquisition apparatus 1, and is basically executed with respect to the received image.

When pre-processing of the image of the subject is completed, the controller 7 executes first post-processing of the image, pre-processing of which is completed (Operation 32).

Pre-processing and first post-processing are image processing basically executed to improve the quality of the received image, and correspond to the above-described first image processing.

When first post-processing is completed, the controller 7 displays the image processed by executing the first post-processing in the second region 12 of the user interface (Operation 33).

Further, when pre-processing of the image of the subject is completed, the controller 7 executes second post-processing of the image, pre-processing of which is completed (Operation 34).

As described above, the controller 7 processes the image, pre-processing of which is completed, by executing various kinds of post-processing and adjusting various effects, such as brightness, contrast, sharpness, saturation, film-effect, window/level, etc. Therefore, images exhibiting different effects are generated according to types of applied post-processing.

When second post-processing is completed, the controller 7 generates thumbnail pictures of the respective images and displays the generated thumbnail pictures in the first region 13 of the user interface (Operation 35). Operations 34 and 35 are executed simultaneously with Operations 32 and 33. Only the thumbnail pictures may be displayed, or the thumbnail pictures together with texts representing features of the kinds of post-processing may be displayed.

The image to which first post-processing is applied is displayed in the second region 12, and the thumbnail pictures of the images to which kinds of second post-processing are applied are displayed in the first region 13. When instructions to a thumbnail picture displayed in the first region 13 are input (Operation 36), the controller 7 magnifies a processed image represented by the thumbnail picture and displays the processed image in the second region 12 (Operation 37).

The instructions to the thumbnail picture may be input by touching the thumbnail picture if the display unit 8 employs a touchscreen, or be input by clicking the thumbnail picture through the input unit 6 including a remote controller, a mouse, a keyboard, a speech recognition device or a motion recognition device.

When the thumbnail picture is touched or clicked in this way, the processed image represented by the thumbnail picture is magnified and displayed in the second region 12. For example, when a user touches or clicks the thumbnail picture of an image formed by changing contrast from among the several thumbnail pictures displayed in the first region 13, the image represented by the corresponding thumbnail picture is magnified and displayed in the second region 12. Here, magnification does not mean exaggeration of a specific part but means fitting of the image represented by the thumbnail to the size of the second region 12.

The medical apparatus according to an aspect of an exemplary embodiment provides the user interface displaying thumbnail pictures of images to which various types of post-processing are applied in advance. When a user desires to apply a desired effect to an image displayed on the display unit 8, the user may process the image by touching or clicking a thumbnail picture exhibiting the desired effect from among various thumbnail pictures in which various types of post-processing are executed, without direct change of parameters or direct input of values. Since an image exhibiting the desired effect may be intuitively recognized and selected through the thumbnail picture, the user may more easily and simply process the image.

As is apparent from the above description, in a medical apparatus and an image display method using the same according to an aspect of an exemplary embodiment, various post-processing results of an image may be intuitively recognized and selected. Therefore, a user may more rapidly and conveniently process the image and thus make a more correct clinical diagnosis.

Although a few exemplary embodiments have been shown and described, it would be appreciated by those skilled in the art that changes may be made in the exemplary embodiments without departing from the principles of the inventive concept, the scope of which is defined in the claims includes equivalents of specific features in the above embodiment description and of the claims.

## Claims

1. An apparatus configured to process medical images, comprising:
- a receiver which receives an image of an inside of a subject, which image is obtained with a medical image acquisition apparatus from a group comprising: computer tomography (CT) apparatus; a magnetic resonance imager (MRI); an X-ray apparatus; and an ultrasonic apparatus;
- a controller, which processes the image through multiple predetermined types of image processing, when the image of the subject is received, and generates objects corresponding to the processed image; and
- a display (8), which displays generated objects corresponding to the processed image,
wherein the controller pre-processes the image to remove artifacts or errors generated due to a physical structure of the image acquisition apparatus, and post-processes the image resulting from having undergone pre-processing separately through a first type, basic image post-processing and simultaneously through second types of image post-processing to improve the quality of the image having undergone pre-processing, and generates multiple objects (17) corresponding to the respective images processed through the second types of image post-processing, and wherein the display (8) is controlled to display the multiple objects (17) in a first region (13) thereof and the first type, basic post-processed image on a second region (12) of the display (8), wherein the controller further accepts user input, such as a touch or click, of a selected one of the multiple objects (17) for magnified display in the second region (12) of the corresponding processed image represented by the selected one of the multiple objects (17), selects second types of image post-processing from all second types of image post-processing, which a user often uses, by calculating the use frequencies of the second types of image post-processing selected by the user, and forms the multiple objects (17) of the selected second types of image post-processing.

2. The apparatus according to claim 1, wherein, in response to inputting instructions into the object, the controller magnifies and displays the processed image represented by the object on the display, and either
- comprising further at least one input from among a group of inputs, comprising: a remote controller, a mouse, a keyboard, a speech recognition device, and a motion recognition device, wherein the instructions input into the object are input through the input, or
- wherein the display comprises a touch screen, and the instructions input into the object are input by touching the object displayed on the display.

3. The apparatus according to claim 2, wherein, when an object picture is touched or clicked, a processed image represented by the object is magnified and displayed enlarged in the display, where a window 22 displaying an image before it is changed into a processed image is provided in the display, and when the window 22 is touched or clicked, the image before the change into the processed image may be displayed again to allow the image before the change and the image after the change to be compared and converted directly therebetween.

4. The apparatus according to claim 1, 2 or 3, wherein the object comprises a thumbnail picture of the image processed through the multiple predetermined types of image processing or text representing features of the multiple predetermined types of image processing.

5. A control method of an apparatus configured to process medical images, the method comprising:
receiving an image of an inside of a subject, which image is obtained with a medical image acquisition apparatus from a group comprising: computer tomography (CT) apparatus; a magnetic resonance imager (MRI); an X-ray apparatus; and an ultrasonic apparatus;
processing the image through multiple predetermined types of image processing, when the image of the subject is received; and
generating an object corresponding to the image processed through the multiple types of image processing and
displaying an object corresponding to the processed image on a display (8) of the medical apparatus,
further comprising pre-processing the image to remove artifacts or errors generated due to a physical structure of the medical image acquisition apparatus, and post-processing the image resulting from having undergone pre-processing separately through a first type, basic image post-processing and simultaneously through second types of image post-processing to improve the quality of the image having undergone pre-processing, generating multiple objects (17) corresponding to the respective images processed through the second types of image post-processing, and displaying the multiple objects (17) in a first region (13) of the display and the first type, basic post-processed image on a second region (12) of the display (8), accepting user input, such as a touch or click, of a selected one of the multiple objects (17) for magnified display in the second region (12) of the corresponding processed image represented by the selected one of the multiple objects (17) and selecting second types of image post-processing from all second types of image post-processing, which a user often uses, by calculating the use frequencies of the second types of image post-processing selected by the user, and forming the multiple objects (17) of the selected second types of image post-processing.

6. The control method according to claim 5, wherein, in response to inputting instructions into the object, the processed image represented by the object is magnified and displayed on the display, wherein either:
- the medical apparatus comprises an input comprising at least one from among a remote controller, a mouse, a keyboard, a speech recognition device, and a motion recognition device, and the instructions input into the object are input through the input, or
- the display comprises a touchscreen, and the instructions input into the object are input by touching the object displayed on the display.

7. The control method according to claim 5 or 6, wherein the object comprises a thumbnail picture of the image processed through the multiple predetermined types of image processing or text representing features of the multiple predetermined types of image processing.

8. The control method according to claim 5, 6 or 7, wherein, when the image of the subject is received, the image of the subject processed through the first type of signal processing is displayed.

9. The control method according to any one of the preceding claims 5 - 8, wherein the object together with the image of the subject processed through the first type of signal processing are displayed on the display of the medical apparatus.

10. The control method according to any one of the preceding claims 5 - 9, wherein the processing of the image through multiple predetermined types of image processing, when the image of the subject is received, comprises:
processing the image, when the image of the subject is received; and
processing the image through the multiple predetermined types of image processing, separately from the processing of the image.

11. The control method according to any one of preceding claims 5 - 10, further comprising:
displaying the object corresponding to the processed image on the display, during the processing of the image separately from the processing of the image.

12. The control method according to any of preceding claims 5 - 11, wherein the post-processing adjusts at least one of parameters of the image having undergone pre-processing, including brightness, contrast, sharpness, saturation, film-effect and window/level.

13. A non-transitory computer readable recording medium comprising a program implementing the control method according to any one of the preceding claims 5 - 12.

## Patentansprüche

1. Vorrichtung, die zum Verarbeiten von medizinischen Bildern konfiguriert ist, wobei die Vorrichtung umfasst:
- einen Empfänger, der ein Bild von dem Inneren eines Subjekts empfängt, wobei das Bild erhalten wurde mit Hilfe einer Vorrichtung zur Erfassung von medizinischen Bildern aus der folgenden Gruppe: eine Computertomographievorrichtung (CT-Vorrichtung); eine Magnetresonanztomographieeinrichtung (MRT); eine Röntgenvorrichtung; und eine Ultraschallvorrichtung;
- eine Steuerung, welche das Bild durch mehrere vorbestimmte Arten von Bildverarbeitung verarbeitet, wenn das Bild des Subjekts empfangen worden ist, und welche Objekte erzeugt, die dem verarbeiteten Bild entsprechen; und
- eine Anzeige (8), welche die erzeugten, dem verarbeiteten Bild entsprechenden Objekte anzeigt, wobei die Steuerung das Bild vor-verarbeitet, um Artefakte und Fehler, die aufgrund einer physikalischen Struktur der Bilderfassungsvorrichtung erzeugt wurden, zu entfernen, und dann das sich aus der Vor-Verarbeitung ergebende Bild mit Hilfe einer ersten, grundlegenden Art von Bild-Nachverarbeitung und gleichzeitig mit Hilfe zweiter Arten von Bild-Nachverarbeitung separat nachverarbeitet, um die Qualität des vorverarbeiteten Bildes zu verbessern, und mehrere Objekte (17) erzeugt, die den jeweiligen, durch die zweiten Arten von Bild-Nachverarbeitung verarbeiteten Bildern entsprechen,
und wobei die Anzeige (8) derart gesteuert wird, dass sie die mehreren Objekte (17) in einem ersten Bereich (13) davon anzeigt, und die mit Hilfe der ersten, grundlegenden Art von Bild-Nachverarbeitung verarbeiteten Bilder in einem zweiten Bereich (12) der Anzeige (8) anzeigt, wobei die Steuerung weiterhin eine Benutzereingabe wie zum Beispiel eine Berührung oder einen Klick auf ein ausgewähltes aus der Mehrzahl von Objekten (17) akzeptiert für eine vergrößerte Anzeige in dem zweiten Bereich (12) des entsprechenden verarbeiteten Bildes, das durch eines der mehreren Objekte (17) repräsentiert wird, dann aus allen zweiten Arten von Bild-Nachverarbeitung die zweiten Arten von Bild-Nachverarbeitung auswählt, die ein Benutzer häufig verwendet, durch Berechnen der Häufigkeit einer Verwendung der von dem Benutzer ausgewählten zweiten Arten von Bild-Nachverarbeitung, und anschließend die mehreren Objekte (17) aus den ausgewählten zweiten Arten von Bild-Nachverarbeitung bildet.

2. Vorrichtung gemäß Anspruch 1, wobei die Steuerung als Reaktion auf eine Eingabe von Anweisungen in das Objekt das verarbeitete Bild, das durch das Objekt auf der Anzeige repräsentiert wird, vergrößert und anzeigt, und wobei entweder
- die medizinische Vorrichtung weiterhin wenigstens eine Eingabeeinrichtung aus einer Gruppe von Eingabeeinrichtungen umfasst, welche umfasst: eine Fernbedienung, eine Computermaus, eine Tastatur, eine Spracherkennungseinrichtung und eine Bewegungserkennungseinrichtung, wobei die in das Objekt eingegebenen Anweisungen durch diese Eingabeeinrichtung eingegeben werden, oder
- die Anzeige einen Berührungsbildschirm umfasst und die in das Objekt eingegebenen Anweisungen durch Berühren des auf der Anzeige angezeigten Objekts eingegeben werden.

3. Vorrichtung gemäß Anspruch 2, wobei, wenn eine Objektdarstellung berührt oder angeklickt wird, ein durch das Objekt repräsentiertes verarbeitetes Bild vergrößert wird und vergrößert auf der Anzeige angezeigt wird, wo ein Fenster (22) zur Verfügung gestellt ist, das ein Bild anzeigt, bevor es in ein verarbeitetes Bild geändert wird, und wenn das Fenster (22) berührt oder angeklickt wird, das Bild vor der Änderung in ein verarbeitetes Bild erneut angezeigt werden kann, damit das unveränderte Bild und das veränderte Bild miteinander verglichen werden können und direkt dazwischen gewechselt werden kann.

4. Vorrichtung gemäß Anspruch 1, 2 oder 3, wobei das Objekt ein Miniaturbild des durch mehrere vorbestimmte Arten von Bildverarbeitung verarbeiteten Bildes oder einen Text, der die Eigenschaften der mehreren vorbestimmten Arten von Bildverarbeitung repräsentiert, umfasst.

5. Steuerungsverfahren einer Vorrichtung, die zum Verarbeiten von medizinischen Bildern konfiguriert ist, wobei das Verfahren umfasst:
Empfangen eines Bildes von dem Inneren eines Subjekts, wobei das Bild erhalten wurde mit Hilfe einer Vorrichtung zur Erfassung von medizinischen Bildern aus der folgenden Gruppe: eine Computertomographievorrichtung (CT-Vorrichtung); eine Magnetresonanztomographieeinrichtung (MRT); eine Röntgenvorrichtung; und eine Ultraschallvorrichtung;
Verarbeiten des Bildes durch mehrere vorbestimmte Arten von Bildverarbeitung, wenn das Bild des Subjekts empfangen worden ist, und Erzeugen eines Objekts, das dem durch mehrere vorbestimmte Arten von Bildverarbeitung verarbeiteten Bild entspricht; und
Anzeigen des Objektes, das dem verarbeiteten Bild entspricht, auf einer Anzeige (8) der medizinischen Vorrichtung,
weiterhin umfassend ein Vorverarbeiten des Bildes, um Artefakte und Fehler, die aufgrund einer physikalischen Struktur der Vorrichtung zur Erfassung von medizinischen Bildern erzeugt wurden, zu entfernen, und dann ein separates Nachverarbeiten des sich aus der Vor-Verarbeitung ergebenden Bildes mit Hilfe einer ersten, grundlegenden Art von Bild-Nachverarbeitung und gleichzeitig mit Hilfe zweiter Arten von Bild-Nachverarbeitung, um die Qualität des vorverarbeiteten Bildes zu verbessern, und Erzeugen mehrerer Objekte (17), die den jeweiligen, durch die zweiten Arten von Bild-Nachverarbeitung verarbeiteten Bildern entsprechen, und Anzeigen der mehreren Objekte (17) in einem ersten Bereich (13) der Anzeige, und der mit Hilfe der ersten, grundlegenden Art von Bild-Nachverarbeitung verarbeiteten Bilder in einem zweiten Bereich (12) der Anzeige (8), Akzeptieren einer Benutzereingabe wie zum Beispiel einer Berührung oder eines Klicks auf ein ausgewähltes aus der Mehrzahl von Objekten (17) für eine vergrößerte Anzeige in dem zweiten Bereich (12) des entsprechenden verarbeiteten Bildes, das durch eines der mehreren Objekte (17) repräsentiert wird, und Auswählen der zweiten Arten von Bild-Nachverarbeitung aus allen zweiten Arten von Bild-Nachverarbeitung, die ein Benutzer häufig verwendet, durch Berechnen der Häufigkeit einer Verwendung der von dem Benutzer ausgewählten zweiten Arten von Bild-Nachverarbeitung, und Bilden der mehreren Objekte (17) aus den ausgewählten zweiten Arten von Bild-Nachverarbeitung.

6. Steuerungsverfahren gemäß Anspruch 5, wobei als Reaktion auf eine Eingabe von Anweisungen in das Objekt das verarbeitete Bild, das durch das Objekt auf der Anzeige repräsentiert wird, vergrößert und auf der Anzeige anzeigt wird, und entweder:
- umfasst die medizinische Vorrichtung wenigstens eine Eingabeeinrichtung aus einer Gruppe von Eingabeeinrichtungen, die umfasst: eine Fernbedienung, eine Computermaus, eine Tastatur, eine Spracherkennungseinrichtung und eine Bewegungserkennungseinrichtung, wobei die in das Objekt eingegebenen Anweisungen durch diese Eingabeeinrichtung eingegeben werden, oder
- die Anzeige umfasst einen Berührungsbildschirm und die in das Objekt eingegebenen Anweisungen werden durch Berühren des auf der Anzeige angezeigten Objekts eingegeben.

7. Steuerungsverfahren gemäß Anspruch 5 oder 6, wobei das Objekt ein Miniaturbild des durch mehrere vorbestimmte Arten von Bildverarbeitung verarbeiteten Bildes oder einen Text, der die Eigenschaften der mehreren vorbestimmten Arten von Bildverarbeitung repräsentiert, umfasst.

8. Steuerungsverfahren gemäß Anspruch 5, 6 oder 7, wobei, wenn das Bild des Objekts empfangen wird, das mit Hilfe der ersten Art von Bildverarbeitung verarbeitete Bild des Objekts angezeigt wird.

9. Steuerungsverfahren gemäß einem der vorangegangenen Ansprüche 5 bis 8, wobei das Objekt gemeinsam mit dem mit Hilfe der ersten Art von Bildverarbeitung verarbeiteten Bild des Subjekts auf der Anzeige der medizinischen Vorrichtung angezeigt wird.

10. Steuerungsverfahren gemäß einem der vorangegangenen Ansprüche 5 bis 9, wobei das Verarbeiten des Bildes mit Hilfe mehrerer vorbestimmter Arten von Bildverarbeitung, wenn das Bild des Subjekts empfangen wird, umfasst:
Verarbeiten des Bildes, wenn das Bild des Subjekts empfangen wird; und
Verarbeiten des Bildes mit Hilfe der mehreren vorbestimmten Arten von Bildverarbeitung, separat von der Verarbeitung des Bildes.

11. Steuerungsverfahren gemäß einem der vorangegangenen Ansprüche 5 bis 10, wobei das Verfahren weiterhin umfasst:
Anzeigen des Objekts, das dem verarbeiteten Bild entspricht, auf der Anzeige während der Verarbeitung des Bildes, separat von der Verarbeitung des Bildes.

12. Steuerungsverfahren gemäß einem der vorangegangenen Ansprüche 5 bis 11, wobei die NachVerarbeitung wenigstens einen Parameter des Bildes, der vorverarbeitet worden ist, anpasst, einschließlich Helligkeit, Kontrast, Schärfe, Sättigung, Filmeffekt und Fenster/Ebene.

13. Übergangsloses computerlesbares Aufzeichnungsmedium, das ein Programm zur Umsetzung des Steuerungsverfahrens gemäß einem der vorangegangenen Ansprüche 5 bis 12 umfasst.

## Revendications

1. Appareil conçu pour traiter des images médicales, comprenant :
- un récepteur qui reçoit une image de l'intérieur d'un sujet, laquelle image est obtenue à l'aide d'un appareil d'imagerie médicale parmi le groupe constitué par : un appareil de tomodensitométrie (TDM), un appareil d'imagerie par résonance magnétique (IRM), un appareil de radiographie et un appareil à ultrasons ;
- un organe de commande, qui traite l'image par le biais de multiples types de traitement d'image prédéterminés, une fois l'image du sujet reçue, et qui produit des objets qui correspondent à l'image traitée ; et
- un dispositif d'affichage (8), qui affiche les objets produits qui correspondent à l'image traitée ; ledit organe de commande prétraitant l'image afin d'éliminer les artefacts ou erreurs découlant de la structure physique de l'appareil d'imagerie, post-traitant l'image résultant de l'opération de prétraitement séparément par le biais d'un post-traitement d'image de base d'un premier type et simultanément par des seconds types de post-traitement d'image pour améliorer la qualité de l'image ayant subi le prétraitement, et produisant des objets multiples (17) qui correspondent aux images respectives traitées par le biais des seconds types de post-traitement d'image,
et le dispositif d'affichage (8) étant commandé pour afficher les objets multiples (17) dans une première zone (13) de celui-ci et l'image ayant subi le post-traitement de base du premier type dans une seconde zone (12) du dispositif d'affichage (8), ledit organe de commande acceptant en outre une saisie réalisée par l'utilisateur, sous la forme d'une entrée tactile ou d'un clic, concernant un objet choisi parmi les objets multiples (17) afin qu'il soit affiché grossi dans la seconde zone (12) de l'image traitée correspondante représentée par l'objet choisi parmi les objets multiples (17), choisissant, parmi tous les seconds types de post-traitement d'image, les seconds types de post-traitement d'image qu'un utilisateur utilise fréquemment, en calculant les fréquences d'usage des seconds types de post-traitement d'image choisis par l'utilisateur, et formant les objets multiples (17) des seconds types de post-traitement d'image choisis.

2. Appareil selon la revendication 1, dans lequel, en réaction à la saisie d'instructions dans l'objet, l'organe de commande grossit et affiche l'image traitée représentée par l'objet sur le dispositif d'affichage, et dans lequel
- soit l'appareil médical comprend en outre au moins un dispositif de saisie appartenant à un groupe comprenant : un organe de commande à distance, une souris, un clavier, un dispositif de reconnaissance vocale, et un dispositif de reconnaissance de mouvement, les instructions saisies dans l'objet étant saisies par le biais du dispositif de saisie,
- soit le dispositif d'affichage comprend un écran tactile, et les instructions saisies dans l'objet sont saisies en touchant l'objet affiché sur le dispositif d'affichage.

3. Appareil selon la revendication 2, dans lequel, lorsqu'une image d'objet subit un contact tactile ou un clic, une image traitée représentée par l'objet est grossie et affichée agrandie sur le dispositif d'affichage, sur lequel est prévu une fenêtre (22) affichant une image non encore modifiée en image traitée, et lorsque la fenêtre (22) subit un contact tactile ou un clic, l'image non encore modifiée en ladite image traitée peut être affichée de nouveau pour permettre de comparer et de transformer directement entre elles l'image non encore modifiée et l'image modifiée.

4. Appareil selon la revendication 1, 2 ou 3, dans lequel l'objet comprend une vignette de l'image traitée par les multiples types de traitement d'image prédéterminés ou du texte représentant les caractéristiques des multiples types de traitement d'image prédéterminés.

5. Procédé de commande d'un appareil conçu pour traiter des images médicales, le procédé comprenant :
la réception d'une image de l'intérieur d'un sujet, laquelle image est obtenue à l'aide d'un appareil d'imagerie médicale parmi le groupe constitué par : un appareil de tomodensitométrie (TDM), un appareil d'imagerie par résonance magnétique (IRM), un appareil de radiographie, et un appareil à ultrasons ;
le traitement de l'image par le biais de multiples types de traitement d'image prédéterminés, une fois l'image du sujet reçue ; et la production d'un objet qui correspond à l'image traitée par le biais des types multiples de traitement d'image ; et
l'affichage d'un objet qui correspond à l'image traitée, sur un dispositif d'affichage (8) de l'appareil médical ;
comprenant en outre le prétraitement de l'image afin d'éliminer les artefacts ou erreurs découlant de la structure physique de l'appareil d'imagerie médicale, et le post-traitement de l'image résultant de l'opération de prétraitement séparément par le biais d'un post-traitement d'image de base d'un premier type et simultanément par des seconds types de post-traitement d'image pour améliorer la qualité de l'image ayant subi le prétraitement, la production d'objets multiples (17) qui correspondent aux images respectives traitées par le biais des seconds types de post-traitement d'image, et l'affichage des objets multiples (17) dans une première zone (13) du dispositif d'affichage et de l'image ayant subi le post-traitement de base du premier type dans une seconde zone (12) du dispositif d'affichage (8), l'acceptation d'une saisie réalisée par l'utilisateur, sous la forme d'une entrée tactile ou d'un clic, concernant un certain objet parmi les objets multiples (17) afin qu'il soit affiché grossi dans la seconde zone (12) de l'image traitée correspondante représentée par l'objet choisi parmi les objets multiples (17) et le choix, parmi tous les seconds types de post-traitement d'image, des seconds types de post-traitement d'image qu'un utilisateur utilise fréquemment, en calculant les fréquences d'usage des seconds types de post-traitement d'image choisis par l'utilisateur, et la formation des objets multiples (17) des seconds types de post-traitement d'image choisis.

6. Procédé de commande selon la revendication 5, dans lequel, en réaction à la saisie d'instructions dans l'objet, l'image traitée représentée par l'objet est grossie et affichée sur le dispositif d'affichage, et dans lequel :
- soit l'appareil médical comprend au moins un dispositif de saisie appartenant à un groupe comprenant : un organe de commande à distance, une souris, un clavier, un dispositif de reconnaissance vocale, et un dispositif de reconnaissance de mouvement, et les instructions saisies dans l'objet sont saisies par le biais du dispositif de saisie,
- soit le dispositif d'affichage comprend un écran tactile, et les instructions saisies dans l'objet sont saisies en touchant l'objet affiché sur le dispositif d'affichage.

7. Procédé de commande selon la revendication 5 ou 6, dans lequel l'objet comprend une vignette de l'image traitée par les multiples types de traitement d'image prédéterminés ou du texte représentant les caractéristiques des multiples types de traitement d'image prédéterminés.

8. Procédé de commande selon la revendication 5, 6 ou 7, dans lequel, une fois l'image du sujet reçue, l'image du sujet traitée par le biais du premier type de traitement d'image est affichée.

9. Procédé de commande selon l'une quelconque des revendications précédentes 5 à 8, dans lequel l'objet ainsi que l'image du sujet traitée par le biais du premier type de traitement d'image sont affichés sur le dispositif d'affichage de l'appareil médical.

10. Procédé de commande selon l'une quelconque des revendications précédentes 5 à 9, dans lequel le traitement de l'image par le biais de multiples types de traitement d'image prédéterminés, une fois l'image du sujet reçue, comprend :
le traitement de l'image, une fois l'image du sujet reçue, et
le traitement de l'image par le biais des multiples types de traitement d'image prédéterminés, séparément du traitement de l'image.

11. Procédé de commande selon l'une quelconque des revendications précédentes 5 à 10, comprenant en outre :
l'affichage de l'objet qui correspond à l'image traitée sur le dispositif d'affichage, pendant le traitement de l'image séparément du traitement de l'image.

12. Procédé de commande selon l'une quelconque des revendications précédentes 5 à 11, dans lequel le post-traitement ajuste au moins un des paramètres de l'image ayant subi le prétraitement, parmi lesquels la luminosité, le contraste, la netteté, la saturation, les effets sur le cliché et le niveau de fenêtre.

13. Support d'enregistrement non transitoire lisible par voie informatique comprenant un programme mettant en oeuvre le procédé de commande selon l'une quelconque des revendications précédentes 5 à 12.
